# EUROPEAN PATENT SPECIFICATION

(11) **EP 0 307 175 B1**
(45) Date of publication and mention of the grant of the patent: **22.03.1995**
(21) Application number: 88308254.7
(22) Date of filing: 07.09.1988
(51) Int. Cl.: G01N 33/531, G01N 33/546, G01N 33/78

(54) **Method for preparing phosphodiester conjugates useful for preparing immunoactive liposomes**
Verfahren zur Herstellung von phosphodiester-Konjugaten, die bei der Herstellung der immunaktiven Liposomen verwendbar sind
Méthode pour la préparation de conjugués de diesters phosphoniques utiles dans la préparation de liposomes immuno-actifs

(30) Priority: 09.09.1987 US 94667
(43) Date of publication of application: 15.03.1989
(73) Proprietor: Ciba Corning Diagnostics Corp., Medfield Massachusetts 02052 (US)
(72) Inventor: Law, Say-Jong, Westwood, MA 02090 (US)
(74) Representative: Froud, Clive

(56) References cited:
- EP-A- 0 144 084
- EP-A- 0 276 165
- EP-A- 0 278 116
- WO-A-86/00142
- US-A- 2 447 715
- US-A- 4 743 560

## Description

This invention relates to a method for preparing phosphodiester conjugates useful for preparing immunoactive liposomes; more particularly, it relates to a method for preparing certain phosphodiester conjugates from certain phospholipids, ligands and analytes, as well as to the preparation of immunoactive liposomes from such conjugates.

Liposome immunoassay utilizes immunoactive liposomes (i.e. liposomes with covalently attached antigen, antibody or other analyte of interest). Preparation of the immunoactive liposomes involves preparing analyte derivatives of phosphatidylethanolamine (PE) or other phospholipids and subsequently incorporating specific quantities of this phospholipid-analyte conjugate into the liposome surface membrane during the formation of the liposome vesicle.

U.S. Patent No. 4,480,041 describes a method for preparing immunoactive liposomes utilizing phosphotriester intermediates of phospholipids derivatized with an analyte. Synthesis of the phosphotriester intermediates and conversion to the targeted phospholipid-analyte derivative is a five or six step process.

U.S. Patent No. 4,193,983 describes the preparation of a conjugate of a carboxylate-containing phenobarbitol and dipalmitoyl phosphatidyl ethanolamine (DPPE) by (1) reacting the phenobarbitol with oxalic acid and then (2) reacting the resultant reaction product with DPPE to produce the conjugate. This patent additionally describes the derivatization of cardiolipin to a cardiolipin hemisuccinate through the reaction of the hydroxyl group of the glycerol moiety in the cardiolipin with succinic anhydride. This hemisuccinate is then activated by oxalyl chloride and subsequently reacted with fluoresceinamine to produce a cardiolipin-succinate-fluorescein conjugate.

In O'Connell et al, "A highly Sensitive Immunoassay System Involving Antibody-Coated Tubes and Liposomes-Entrapped Dye", Clin. Chem. 31\9, 1424-1426 (1985), the authors describe the direct coupling of DPPE and 3-Ketodigoxigenin to form a Schiff base which was then reduced with sodium cyanogenborohydride to produce a DPPE-digoxigenin conjugate.

In Haga et al, "Drug Sensor: Liposome Immunosensor for Theophylline", Anal. Biochem. 118, 286-293 (1981), the authors describe a process for the preparation of a theophylline-phosphatidylethanolamine (PE) conjugate by (1) reacting theophylline and thionyl chloride to produce an acid chloride of theophylline (a theophylline analog with a carboxylate group); and then (2) reacting the acid chloride of theophylline with PE to produce the theophylline-PE conjugate.

In EP-A-144 084, there is disclosed incorporating a phospholipid-ligand composite into liposomes, followed by coupling an immunoactive partner to the liposome via the remaining functional group of the phospholipid-ligand composite situated on the surface of the liposome. Similarly, in EP-A-276 165, (which claims an earlier priority date, but is not a prior publication), the phospholipid is reacted with a ligand, formed to a liposome and thereafter reacted with an analyte.

In WO-86/00142, there is disclosed an immunoliposome assay wherein a ligand-lipid complex is employed to stabilize an otherwise unstable liposome composition.

It is the object of the present invention to provide a novel method of preparing phosphodiester-ligand-analyte conjugates useful in preparing immunoactive liposomes.

This invention relates to a method for the preparation of an analyte-functionalized liposome characterised in that it comprises:
(a) reacting a polar phospholipid corresponding to the following general formula: 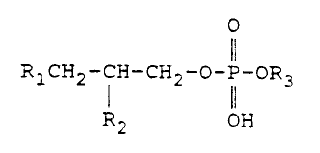 wherein R₁ and R₂ independently represent hydrogen, hydroxyl, R˝, OR˝ or 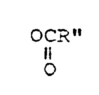 wherein R˝ represents a saturated or unsaturated, branched- or straight-chain alkyl or alkylene group having from 1 to 24 carbon atoms and wherein at least one of R₁ and R₂ represents 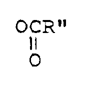 or OR˝, while the other independently represents hydrogen, hydroxyl, R˝, OR˝, or 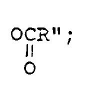 and wherein R₃ represents a branched- or straight-chain alkyl amino alkyl group from 1 to 24 carbon atoms; with a ligand by direct derivatization to produce a polar phospholipid-ligand composite;
(b) reacting the polar phospholipid-ligand composite, which may be previously activated, with an analyte, to produce the phosphodiester-ligand-analyte conjugate;
   the ligand being reactive with the R₃ moiety of the phospholipid and reactive with the analyte; and
(c) forming an analyte-functionalized liposome with the phosphodiester-ligand-analyte conjugate, which is functionalized on its surface with the analyte and has a marker carried within the liposome.

The present invention also relates to method of forming an analyte-functionalized liposome for use in immunoassays characterised in that it comprises:
(a) preparing a phosphodiester-ligand-analyte conjugate by such a method;
(b) adding a marker to the dispersion containing the conjugate; and
(c) forming therefrom an analyte-functionalized liposome which is functionalized on its surface with the analyte and has a marker carried within the liposome.

The phosphodiester-ligand-analyte conjugate may then be purified, if desired.

Preferably, R₁ and R₂ are 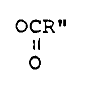
or OR˝; R₃ is a straight-chain amino alkyl group of from 2 to 4 carbon atoms, and R" is a saturated straight-chain alkyl of from 10 to 20 carbon atoms. More preferably, R₁ and R₂ are 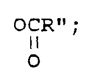
R₃ is aminoethyl; and R˝ is a saturated straight-chain alkyl of 15 carbon atoms.

The ligand useful in the process of this invention acts as a connector, or spacer, between the phospholipid and the analyte, i.e., the ligand is a bifunctional compound which enables the attachment at one end to the desired analyte and at the other end to the phospholipid. The ligands are preferably alkyl groups of from 2 to 20 carbon atoms, branched or unbranched, which are optionally substituted with 0-20 heteroatoms, normally nitrogen, oxygen, sulfur, or phosphorus, and two distinct functional groups comprising carboxyl, amine, hydroxyl, sulfhydryl, imidate, maleimide, 2-pyridylthio, halide, isothiocyanate, or anhydride residues, preferably carboxyl, amine, hydroxyl or sulfhydryl residues, preferably at the opposite ends of the ligand. One end of the ligand is designed for attachment to the phospholipid, preferably via a carboxyl group. The actual mechanism for attachment of the ligands to the phospholipids and analytes will depend on the nature of the reactive components. Certain ligands need to be activated (derivatized to form a reactive group capable of coupling to the phospholipid) prior to reacting the ligand with the phospholipid. Certain phospholipid-ligand composites also need to be activated (derivatizing the ligand of the phospholipid-ligand composite to form a reactive group capable of coupling to the desired analyte) prior to reacting the phospholipid-ligand composite with the analyte. Anhydrides, thionylchloride, sulfonic acid derivatives, N-hydroxysuccinamides, carbodiimides, and other neans of activation (or reactive group formation) known in the art can be used to prepare the ligand for reaction with the phospholipid or prepare the phospholipid-ligand composite for reaction with the analyte. The type of activation (or derivatization) necessary will depend on the particular phospholipid, ligand and analyte combination to be used.

Useful ligands include hetero- and homo- bifunctional cross-linking reagents and cyclic acid anhydrides. Examples of preferred ligands include succinimyl 4(p-maleimidophenyl)-butyrate (SMPB); N-succinimydyl 3(2-pyridyldithio)proprionate; 2-iminothiolane; succinic anhydride, and the like.

The analytes useful in the method of this invention are those materials which are capable of coupling to the ligand of the polar phospholipid-ligand composite. Examples of suitable analytes include antigens, circulating hormones, antibiotics and other theraputic drugs, and derivatives thereof. Useful analytes include folate, digoxin, B-12 and theophylline. Preferred analytes are amino-containing analytes such as thyroxine and triiodothyronine. Certain analytes, such as e.g., folate, digoxin, or B-12, can require activation (derivatization) in order for these analytes to be coupled to the ligand-containing phospholipid. The type of derivatization necessary will depend on the specific analyte-ligand combination to be used.

In a preferred embodiment of the method of this invention, the phospholipid is a phosphatidylethanolamine (PE) compound having the formula: 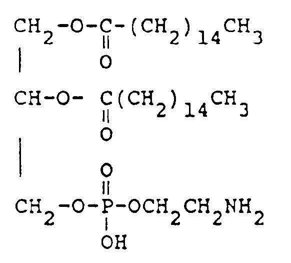
Reaction of the PE with a cyclic anhydride such as succinic anhydride yields a phosphatidyldiester derivative (PE-Suc) having the formula: 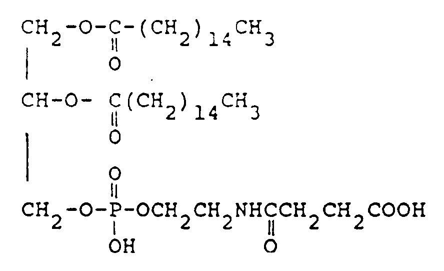
The carboxyl group of the PE-Suc derivative can be activated by reaction with an activating reagent such as ethyl chloroformate (to form a mixed anhydride), dicyclohexylcarbodiimide and N-hydroxysuccinimide (to form an active ester) or oxalyl chloride or thionyl chloride (to form an acyl chloride).

Activation of the PE-Suc derivative by the mixed anhydride method can be conducted in an aprotic solvent, preferably chloroform. The activating reagent can be a branched or straight-chain alkyl chloroformate, preferably the alkyl group is ethyl. Addition of an organic base is required to neutralize any hydrogen chloride generated. This activation is preferably conducted at a temperature of from about -20°C to about 25°C, more preferably from about 0°C to about 10°C, for a period of time from about 5 minutes to about 90 minutes, most preferably from about 20 minutes to about 30 minutes.

The analyte, e.g., thyroxin, is then added in equimolar amounts or in molar excess of the amount of PE-Suc derivative because separation of the desired conjugate (e.g., PE-Suc-T₄) from excessive, unreacted analyte (e.g., T₄) is easier than from unreacted PE-Suc.

Immediately after adding the analyte, a polar, aprotic solvent, e.g., dimethylformamide (DMF), in volume ranging from one-half to three times that of the original reaction mixture should be added. The yield of the desired conjugate is improved by addition of the solvent. After the addition of the solvent, the reaction is kept at room temperature for about 0.5 hours to about 16 hours.

PE-Suc can also be activated by an active ester method using a carbodiimide, such as dicyclohexylcarbodiimide (DCC), and N-hydroxysuccinimide (NHS) in equimolar or excess amounts of the PE-Suc derivative. PE-Suc is initially reacted with DCC in an aprotic, polar or non-polar, preferably non-polar, solvent such as chloroform, at room temperature or preferably, a lower temperature, for about 5 to about 30 minutes, preferably about 10 to about 15 minutes, to form a reaction adduct between PE-Suc and DCC. NHS is then added to this reaction adduct and reacted at room temperature overnight to produce the desired ester, PE-Suc-NHS, which has the following formula: 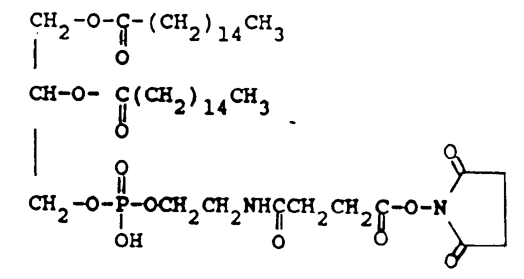
and dicyclohexylurea as a by-product. NHS can be added to the reaction mixture in solid form followed by addition of a polar, aprotic solvent such as DMF. Alternatively, NHS can first be dissolved in the polar solvent and then added to the reaction mixture.

Prior to the addition of the analyte (e.g., thyroxin) the aprotic solvents are removed from the reaction mixture, e.g., by evaporation. The analyte is then added in equimolar or molar excess amounts of the PE-Suc derivative, to the reaction mixture. Immediately, after addition of the analyte, a polar, protic solvent, preferably methanol in the presence of an organic base such as triethylamine, is added in excess. The reaction is then conducted at room temperature overnight to produce the desired conjugate, e.g. PE-Suc-T₄.

To separate the resultant desired conjugate from the reaction mixture, a purification procedure, such as preparative thin layer chromatography (PLC), is utilized.

In a preferred embodiment employing a PLC procedure, the crude reaction mixture containing the desired conjugate is first dissolved in a solvent system of sufficient polarity to facilitate even adsorption of the material throughout the thickness of the silica gel of a preparative thin layer chromatography plate. Preferably, a pre-coated silica gel (60 angstrom pore-sized particles, 2mm thickness) preparative thin layer chromatography plates (20 x 20 cm) with fluorescence indicator is utilized for the PLC purification. The solvent system utilized should be equal to or more polar than a 20% methanol/chloroform solvent system, preferably capable of being enhanced by the addition of water. A preferred solvent system is chloroform/methanol/water in a ratio of 73:24:3, respectively. The crude reaction mixture is loaded onto the plates in amounts of between 125-250 mg/plate, preferably 150-200 mg/plate, in a volume of between 1-4 ml/plate, preferably, between 2-3 ml/plate.

Purification of a preferred conjugate, PE-Suc-T₄, from the crude reaction mixture utilizes an organic acid containing solvent system, such as, for example, chloroform/methanol/acetic acid (60:20:3) which separates PE-Suc-T₄ and unreacted T₄ into two distinct bands on the plate. The PE-Suc-T₄ band is then stripped off the plate and effectively recovered by eluting in a polar solvent system such as, for example,
chloroform/methanol/water (55:40:5).

Frequently, the PE-Suc-T₄ so produced will be contaminated with unreacted PE-Suc. To separate the PE-Suc-T₄ from PE-Suc a non-acid containing polar solvent system is utilized, such as, for example, chloroform/methanol/water (55:40:5), on a second PLC plate on which the eluted band from the first PLC plate is loaded. The PE-Suc-T₄ is stripped and eluted. This PLC step to separate PE-Suc-T₄ from unreacted PE-Suc is repeated until a PE-Suc-T₄/chloroform solution (0.5 mg/ml) exhibits an absorbance at A300 of 1.05 or higher. The PE-Suc-T₄ so produced is then useful for thyroxin-liposome preparation.

Other preparative chromatographic procedures which can be used to separate the desired conjugate prepared by the method of this invention from the crude reaction mixture include low to medium-pressure short column chromatography, flash chromatography and high performance liquid chromatography (HPLC). HPLC, although requiring expensive equipment, can be expected to be a very efficient and simple procedure once optimal elution conditions are determined.

Liposomes incorporating the conjugates prepared according to the method of this invention can be prepared using procedures known in the art. For example, several useful procedures are described in Szoka et al., Ann. Rev. Biophys. Bioeng. 9, pp. 467-508 (1980).

The following Examples are presented to illustrate the method of this invention.

### EXAMPLE 1

### Preparation of N-(3-Carboxypropionyl)(1,2-dipalmitoyl-3-rac-phosphatidyl)ethanolamine (PE-Suc)

A mixture of (1,2-dipalmitoyl-3-rac-phosphatidyl)ethanolamine (PE, 440 mg, 0.637 mmole), succinic anhydride (76 mg, 0.764 mmole), triethylamine (0.09 ml, 0.637 mmole) in 40 ml of DMF:CHCl₃ (1:1) was stirred, heated at 65°C for 1.5 hours, and evaporated in vacuo. The oily residue was taken up with 4 ml of 20% methanol in chloroform and streaked on silica gel preparative TLC plates (Merck & Co., Inc., Rahway, N.J., Catalog No. 5717-7) at the loading ratio of 0.25 g/plate. The plates were developed with chloroform/methanol/water (55:40:5). A faint UV band (Rf = 0.30) was stripped, eluted with chloroform/methanol/water (54:40:5). The eluent was evaporated and the residue triturated with 30 ml of chloroform and filtered. The filtrate was evaporated to give the desired product PE-Suc (470 mg, 93%).

### EXAMPLE 2

### Preparation of N-[3-(α-carboxy-3,3′, 5,5′-tetraiodo-L-thyronyl) propionyl](1,2-dipalmitoyl-3-rac-phosphatidyl)-ethanolamine (PE-Suc-T₄)

### (A) Mixed Anhydride Method:

A solution of PE-Suc (402 mg, 0.508 mmole, from Example 1) in 10 ml of chloroform was treated with triethylamine (100 ul, 0.70 mmole), cooled in an ice-bath and then treated with ethyl chloroformate (65 ul, 0.70 mmole). The reaction mixture was stirred for 20 minutes, treated with L-thyroxin (sodium salt, 406 mg, 0.508 mmole) and 10 ml of dimethylformamide. The ice-bath was removed. A complete solution was obtained in 10-15 minutes. The reaction mixture was stirred at room temperature overnight, coevaporated with 10 ml of xylene 2 times to complete dryness. The desired PE-Suc-T₄ was isolated by repeated PLC as follows: The dried reaction mixture was dissolved in 10 ml of chloroform/methanol/water (73:24:3), streaked on silica gel preparative TLC plates (Merck & Co., Inc., Rahway, N.J., Catalog No. 5717-7) at the loading ratio of 200 mg/plate, and developed with chloroform/methanol/acetic acid (60:20:3). The desired product was identified as a major UV absorbing band (Rf = 0.42) which also gave a positive test with a molybdenum spray (test for organophosphate). The band was stripped, and eluted with 150 ml of chloroform/methanol/water (54:40:5). The residue obtained from the evaporation of the eluent was subjected to a second PLC purification using chloroform/methanol/water (65:25:4) to develop the plates. The desired band was identified, stripped, eluted, and evaporated in the same manner as the first PLC. The residue so obtained was triturated with 30 ml of chloroform and filtered through Whatman® #1 filter paper by gravity. The filtrate was evaporated to give the pure desired product PE-Suc-T₄ (202 mg, 25%).
Anal. Calc for C₅₆H₈₇N₂I₄O₁₄P: C, 43.36; H, 5.65; N, 1.81.
Found: C, 43,51: H, 5.90; N, 1.67.

### (B) Active Ester Method:

A solution of PE-Suc (100 mg, 0.126 mmole, from Example 1) in 5 ml of chloroform was cooled in ice bath, treated with DCC (39 mg, 0.19 mmole) in 1 ml of chloroform. After 10 min, NHS (22 mg, 0.19 mmole) in 1 ml of DMF was added. The reaction was stirred at room temperature overnight, coevaporated with 2 ml of xylene to complete dryness. To the residue was added thyroxin (sodium salt, 150 mg, 0.19 mmole) and triethylamine (90 ul, 0.63 mmole) in 10 ml of methanol. The reaction mixture was stirred at room temperature overnight and evaporated to dryness.
The desired PE-Suc-T₄ (50 mg, 25.6%) was isolated by repeated PLC in the same manner as described in (A) above.

### (C) Active Ester Method:

The same procedure as described in (B) was used except that the desired PE-Suc-T₄ was isolated and purified from the reaction mixture by flash chromatography on a 100-ml glass column (Aldrich Chemical Co., Inc., Milwaukee, WI, Catalog No. Z14, 735-4), packed with a slurry of silica gel (40 um, J.T. Baker Chemical Co., Phillipsburg, N.J., Catalog No. 7024-1), in chloroform/methanol/acetic acid (73:24:3) under 10-15 psi nitrogen. The fractions containing pure PE-Suc-T₄ were pooled, evaporated to dryness. The residue was repurified by PLC using chloroform/methanol/water (55:40:5) to develop the plate. The yield of pure PE-Suc-T₄ was 16.2%.

### EXAMPLE 3

### Preparation of N-[3-(α-carboxy-3,3′,5-triiodo-L-thyronyl)-propionyl](1,2-dipalmitoyl-3-rac-phosphatidyl)ethanolamine (PE-Suc-T₃)

A solution of PE-Suc (360 mg, 0.45 mmole from Example 1) in 7 ml of chloroform was treated with triethylamine (78 ul, 0.54 mmole), cooled in an ice-bath and treated with ethyl chloroformate (54 ul, 0.54 mmole). The reaction was stirred for 25 minutes, treated with T₃ (293 mg, 0.45 mmole) and 7 ml of dimethylformamide. The ice-bath was removed. A complete solution was obtained in 10-15 minutes. The reaction mixture was stirred at room temperature overnight, coevaporated with 10 ml of xylene to complete dryness.
The desired PE-Suc-T₃ (65 mg, 9.5%) was isolated by PLC in the same manner as described in Example 2A above.

### EXAMPLE 4

### Preparation of Immunoactive T₄ Liposomes Using PE-Suc-T₄

### A. Preparation of Lipid Film

A solution of PE-Suc-T₄ (0.95 mg., OD 300 = 2.0/ul of chloroform, from Example 2A), L-dipalmitoyl phosphatidylcholine (DPPC) (125 mg) (Avanti Polar Lipids, Inc., Birmingham, AL), cholesterol (67.5 mg) (Sigma Chemical Co., St. Louis, MO), and L-dipalmitoyl phosphatidylglycerol (11.5 mg) (Avanti Polar Lipids, Inc., Birmingham, AL), in 5 ml of chloroform was evaporated on an 8 cm diameter crystallizing dish (Corning Glass Works, Corning, N.Y.) under vacuo of 6-100 mm Hg at room temperature for 2 hours, then at 10-100 milliTorr overnight (16 hours) to obtain a dry, thin homogeneous lipid coating on the bottom of the dish.

### B. Lipid Vesicle Formation/Enzyme Encapsulation

The dish containing the lipid film prepared in A was prewarmed in an orbital shaker water bath at 38-39°C for 10-12 minutes. A solution of glucose-6-phosphate dehydrogenase (37.5 kU) (Toyobo Biochemicals Co., Osaka, Japan) in 15 ml of 2% glycerol/0.25 mM EDTA, pH7.3, was added to the warmed lipid film in the dish. The dish was sealed with parafilm and shaken at 150-250 rpm at 38-39°C for 20 minutes to form lipid vesicles.

### C. Dialysis

The lipid vesicles prepared in B were allowed to cool to room temperature in the dish, transferred to dialysis tubing using a Pasteur pipet and then dialyzed against 1 liter of a cold Tris Buffer of 0.1M Tris /0.5 mM EDTA/0.03M NaCl/0.01% sodium azide, pH 7.8, at 2-8°C for 2 days with one change of buffer after 24 hours.

### D. Extrusion/Sizing of Lipid Vesicles

The dialyzed lipid vesicles of C were transferred to a beaker and warmed at 30-32°C for 10 minutes in a water bath and then extruded through 25 mm diameter polycarbonate membranes (Nucleopore Corp., Pleasanton, CA) of the following pore sizes and at the respective pressures:
3.0 um - 18 psi
2.0 um - 20 psi
1.0 um - 24 psi
0.6 um - 30 psi
using the Liposome Extruder.

### E. Washing of the Sized Vesicles

The extruded liposome vesicles from D were removed from the extruder and centrifuged at 35K rpm (BECKMAN L5-75, with Type 65 rotor) at 5-10°C for 60 minutes. The clear supernatant was removed and the pellet was resuspended in the Tris Buffer described in C and centrifuged. This step was repeated 3 more times. The resultant washed pellet of liposome vesicles was suspended in 10 ml of the Tris Buffer. The T₄-liposomes so produced were then used in an Enzyme Membrane Immunoassay (EMIA).

## Claims

1. A method for the preparation of an analyte-functionalized liposome characterised in that it comprises:
(a) reacting a polar phospholipid corresponding to the following general formula: 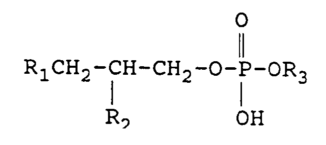 wherein R₁ and R₂ independently represent hydrogen, hydroxyl, R˝, OR˝ or 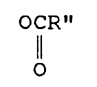 wherein R˝ represents a saturated or unsaturated, branched- or straight-chain alkyl or alkylene group having from 1 to 24 carbon atoms and wherein at least one of R₁ and R₂ represents 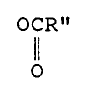 or OR˝, while the other independently represents hydrogen, hydroxyl, R˝, OR˝, or 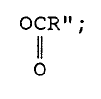 and
wherein R₃ represents a branched- or straight-chain amino alkyl group having from 1 to 24 carbon atoms; with a ligand by direct derivation to produce a polar phospholipid-ligand composite;
(b) reacting the polar phospholipid-ligand composite, which may be previously activated, with an analyte, to produce the phosphodiester-ligand-analyte conjugate;
the ligand being reactive with the R₃ moiety of the phospholipid and reactive with the analyte;
(c) forming therefrom an analyte-functionalized liposome which is functionalized on its surface with the analyte and has a marker carried within the liposome.

2. A method as claimed in claim 1 wherein R₁ and R₂ independently represent 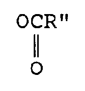 or OR˝; and
R˝ represents a saturated straight-chain alkyl having from 10 to 20 carbon atoms; and R₃ represents a straight-chain aminoalkyl group having from 2 to 4 carbon atoms.

3. A method as claimed in claim 1 or claim 2 wherein the ligand comprises alkyl having from 2 to 20 carbon atoms, branched- or straight-chain, optionally substituted with from 0 to 20 heteroatoms and two distinct functional groups; and/or is a hetero- or homo-bifunctional cross-linking reagent or a cyclic acid anhydride.

4. A method as claimed in any of claims 1 to 3 wherein the analyte is an amino-containing analyte; and/or is thyroxine or triiodothyronine.

5. A method as claimed in any of claims 1 to 4 wherein the phosphodiester-ligand-analyte conjugate is purified by preparative chromatography.

6. A method of forming an analyte-functionalized liposome for use in immunoassays characterised in that it comprises:
(a) preparing a phosphodiester-ligand-analyte conjugate by a method as claimed in any of claims 1 to 5;
(b) adding a marker to the dispersion containing the conjugate; and
(c) forming therefrom an analyte-functionalized liposome which is functionalized on its surface with the analyte and has a marker carried within the liposome.

## Patentansprüche

1. Verfahren zur Herstellung eines analyt-funktionalisierten Liposoms, dadurch gekennzeichnet, daß es umfaßt:
a) Umsetzen eines polaren Phospholipids der nachfolgenden allgemeinen Formel: 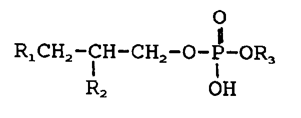 worin R₁ und R₂ unabhängig voneinander Wasserstoff, Hydroxyl, R˝, OR˝ oder 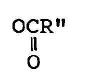 sind,
worin R˝ eine gesättigte oder ungesättigte, verzweigt- oder geradkettige Alkyl- oder Alkylengruppe mit 1 bis 24 Kohlenstoffatomen ist, und worin wenigstens einer der Reste R₁ und R₂ 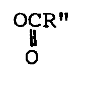 oder OR˝ ist,
während der andere unabhängig vom ersten Wasserstoff, Hydroxyl, R˝, OR˝ oder 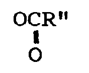 ist; und
worin R₃ eine verzweigt- oder geradkettige Aminoalkylgruppe mit 1 bis 24 Kohlenstoffatomen ist; mit einem Liganden durch direkte Abzweigung, um einen polaren Phospholipid-Liganden-Verbundstoff herzustellen;
b) Umsetzen des polaren Phospholipid-Liganden-Verbundstoffs, der vorher aktiviert worden sein kann, mit einem Analyten, um das Phosphodiester-Liganden-Analyt-Konjugat herzustellen, wobei der Ligand mit dem R₃-Rest des Phospholipids und mit dem Analyten reaktionsfähig ist;
c) Ausbilden eines analyt-funktionalisierten Liposoms hieraus, das auf seiner Oberfläche mit dem Analyten funktionalisiert ist und einen im Liposom angeordneten Marker aufweist.

2. Verfahren nach Anspruch 1, worin R₁ und R₂ unabhängig 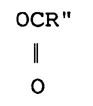 oder OR˝ sind; und
R˝ ein gesättigtes geradkettiges Alkyl mit 10 bis 20 Kohlenstoffatomen ist; und R₃ eine geradkettige Aminoalkylgruppe mit 2 bis 4 Kohlenstoffatomen ist.

3. Verfahren nach Anspruch 1 oder 2, worin der Ligand ein Alkyl mit 2 bis 20 Kohlenstoffatomen, verzweigt- oder geradkettig, wahlweise substituiert mit 0 bis 20 Heteroatomen, und zwei distinkten funktionellen Gruppen aufweist; und/oder ein hetero- oder homo-bifunktionelles Vernetzungsmittel oder ein zyklisches Säureanhydrid ist.

4. Verfahren nach einem der Ansprüche 1 bis 3, worin der Analyt ein Amino enthaltender Analyt ist; und/oder Thyroxin oder Trijodthyronin ist.

5. Verfahren nach einem der Ansprüche 1 bis 4, worin das Phosphodiester-Liganden-Analyt-Konjugat durch präparative Chromatographie gereinigt wird.

6. Verfahren zur Bildung eines analyt-funktionalisierten Liposoms zur Verwendung in Immunoassays, dadurch gekennzeichnet, daß es umfaßt:
a) Herstellen eines Phosphodiester-Liganden-Analyt-Konjugats durch ein Verfahren nach einem der Ansprüche 1 bis 5;
b) Zugeben eines Markers zur das Konjugat enthaltenden Dispersion; und
c) Ausbilden eines analyt-funktionalisierten Liposoms hieraus, das auf seiner Oberfläche mit dem Analyt funktionalisiert ist und einen Marker aufweist, der im Liposom angeordnet ist.

## Revendications

1. Un procédé pour la préparation d'un liposome fonctionnalisé par un analyte, caractérisé en ce qu'il consiste à :
(a) faire réagir un phospholipide polaire correspondant à la formule générale suivante : 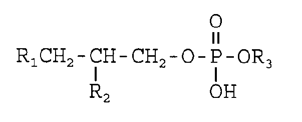 où R₁ et R₂ représentent indépendamment l'hydrogène, un groupe hydroxyle, R˝, OR˝ ou 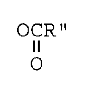 où R˝ représente un groupe alkyle ou alkylène à chaîne droite ou ramifiée, saturé ou insaturé, ayant 1 à 24 atomes de carbone, et où l'un au moins de R₁ et R₂ représente 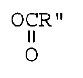 ou OR˝, tandis que l'autre représente indépendamment l'hydrogène, un groupe hydroxyle, R˝, OR˝ ou 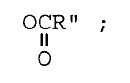 et où R₃ représente un groupe aminoalkyle à chaîne droite ou ramifiée ayant 1 à 24 atomes de carbone ;
avec un ligand par dérivation directe pour produire un composé phospholipide polaire-ligand ;
(b) faire réagir le composé phospholipide polaire-ligand, qui peut être préalablement activé, avec un analyte pour produire le conjugué phosphodiester-ligand-analyte ; le ligand étant réactif avec le fragment R₃ du phospholipide et réactif avec l'analyte ;
(c) former à partir du produit obtenu un liposome fonctionnalisé par un analyte, qui est fonctionnalisé sur sa surface avec l'analyte et comporte un marqueur transporté dans le liposome.

2. Un procédé tel que revendiqué dans la revendication 1, dans lequel R₁ et R₂ représentent indépendamment 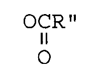 ou OR˝ ; et R˝ représente un groupe alkyle saturé à chaîne droite ayant 10 à 20 atomes de carbone ; et R₃ représente un groupe aminoalkyle à chaîne droite ayant 2 à 4 atomes de carbone.

3. Un procédé tel que revendiqué dans la revendication 1 ou la revendication 2, dans lequel le ligand comprend un groupe alkyle à chaîne droite ou ramifiée ayant 2 à 20 atomes de carbone, facultativement substitué par 0 à 20 hétéroatomes et deux groupes fonctionnels distincts ; et/ou est un réactif hétéro- ou homobifonctionnel formateur de liaison transversale ou un anhydride cyclique d'acide.

4. Un procédé tel que revendiqué dans l'une quelconque des revendications 1 à 3, dans lequel l'analyte est un analyte aminé ; et/ou est la thyroxine ou la triiodothyronine.

5. Un procédé tel que revendiqué dans l'une quelconque des revendications 1 à 4, dans lequel le conjugué phosphodiester-ligand-analyte est purifié par chromatographie préparative.

6. Un procédé de formation d'un liposome fonctionnalisé par un analyte destiné à être utilisé dans des analyses immunologiques, caractérisé en ce qu'il consiste à :
(a) préparer un conjugué phosphodiester-ligand-analyte par un procédé tel que revendiqué dans l'une quelconque des revendications 1 à 5 ;
(b) ajouter un marqueur à la dispersion contenant le conjugué ; et
(c) former à partir du produit résultant un liposome fonctionnalisé par un analyte, qui est fonctionnalisé sur sa surface avec l'analyte et comporte un marqueur transporté dans le liposome.
